# EUROPÄISCHE PATENTSCHRIFT

(11) **EP 4 355 221 B1**
(45) Veröffentlichungstag und Bekanntmachung des Hinweises auf die Patenterteilung: **14.10.2026**
(21) Anmeldenummer: 22737423.8
(22) Anmeldetag: 17.06.2022
(51) Int. Cl.: A61B 10/00, A61B 10/02, A61F 13/38

(54) **ABSTRICHTUPFER FÜR EINE PROBENNAHME**
SWAB FOR COLLECTING A SAMPLE
ÉCOUVILLON DE PRÉLÈVEMENT D'ÉCHANTILLON

(30) Priorität: 18.06.2021 DE 202021103298 U
(43) Veröffentlichungstag der Anmeldung: 24.04.2024
(73) Patentinhaber: Diebold Innovation GmbH, 14165 Berlin (DE)
(72) Erfinder: RIESTER, Markus, 65193 Wiesbaden (DE)
(74) Vertreter: Eisenführ Speiser
(86) Internationale Anmeldenummer: PCT/EP2022/066628
(87) Internationale Veröffentlichungsnummer: WO 2022/263671

(56) Entgegenhaltungen:
- WO-A1-2021/026549
- WO-A1-2021/042098
- US-A1- 2004 071 594
- US-A1- 2007 255 175
- US-B1- 10 064 606

## Beschreibung

Die Erfindung betrifft einen Abstrichtupfer für eine Probennahme.

Abstrichtupfer dienen beispielsweise zum Aufnehmen einer Speichelprobe. Abstrichtupfer haben üblicherweise einen länglichen Schaft, der wenigstens ein Ende aufweist, das für die Probennahme ausgebildet ist. Beispielsweise kann an diesem Ende ein Wattebausch oder ein Schwämmchen oder dergleichen vorgesehen sein. Abstrichtupfer haben daher Ähnlichkeit mit bekannten Wattestäbchen.

Um den Abstrichtupfer für eine Probennahme verwenden zu können, muss der Schaft lang genug sein, um den Abstrichtupfer sicher halten zu können und das der Probennahme dienende Ende an den Entnahmeort zu führen. Der Entnahmeort kann beispielsweise tief im Rachen einer Person sein. Dies erfordert eine Schaftlänge, die erheblich größer ist als die Schaftlänge üblicher Wattestäbchen. Für die Probennahme vorgesehene Abstrichtupfer können daher unhandlich sein.

Aus WO 2021/042098 A1 ist ein Abstrichtupfer mit ausklappbaren Schaft in einer sterilen Hülle beschrieben und gezeigt.

DE 7529837 U zeigt, wie ein Abstrichtupfer nach dessen Gebrauch durch Abbrechen zunächst verkürzt und dann gefaltet werden kann.

US 10,064,606 B1 zeigt ein Gefäß zur Probennahme mit integriertem Teleskop- Abstrichtupfer.

Aus US 2016/0051415 A1 geht ein Abstrichtupfer hervor, dessen distales Ende nach Probennahme abgebrochen und in einem Probengefäß platziert werden kann.

US2004/071594 A1 offenbart einen Abstrichtupfer mit den Merkmalen des Oberbegriffs von Anspruch 11.

Vor diesem Hintergrund ist es ein Ziel der Erfindung, einen handlicheren Abstrichtupfer zu schaffen.

Die Erfindung ist in Anspruch 11 definiert.

Erfindungsgemäß wird dieses Ziel mit einem Abstrichtupfer erreicht, dessen Schaft einen zweiten, für eine Probennahme geeigneten Zustand annimmt, in dem der Schaft länger ist als in einem ersten, zum Lagern oder Transportieren des Abstrichtupfers vorgesehenen Zustand.

Dabei ist der Abstrichtupfer selbst oder in Verbindung mit einer Hülle so ausgeführt, dass der Schaft bei Entnahme aus der Hülle automatisch seinen zweiten, für eine Probennahme geeigneten Zustand annimmt, in dem der Schaft länger ist als in dem ersten, in die Hülle passenden Zustand.

Der Schaft des Abstrichtupfers enthält ein oder mehrere elastische Elemente, beispielsweise ein elastisches Zugelement, das so angeordnet und ausgeführt ist, dass der Schaft selbsttätig seinen zweiten, für die Probeentnahme geeigneten, gestreckten Zustand annimmt. Wenn der Schaft des Abstrichtupfers ein elastisches Biegeelement aufweist, kann der Schaft durch die Hülle in dem ersten, verkürzten Zustand gehalten werden und sich nach Entnahme aus der Hülle selbsttätig entfalten.

Im Falle eines Teleskopschafts kann die Hülle so ausgeführt sein, dass das distale Ende des Abstrichtupfers klemmend gehalten ist, so dass sich der Schaft des Abstrichtupfers durch Zug an dessen proximalen Ende zunächst teleskopartig streckt, bevor dann ein weiterer Zug an dem distalen Ende dazu führt, dass das distale Ende des Schafts aus der klemmenden Halterung und der Hülle gezogen und damit der Abstrichtupfer vollständig aus der Hülle entnommen wird.

Die klemmende Halterung kann dabei durch eine entsprechende Verschweißung oder Verklebung einer Folienverpackung als Hülle für den Abstrichtupfer realisiert sein.

In allen Fällen braucht der Schaft nur an dessen proximalem Ende gegriffen zu werden - und zwar sowohl bei der Entnahme aus der Hülle als auch bei der Benutzung. D.h. ein Berühren des distalen Abschnitts des Abstrichtupfers ist selbst zum Überführen des Abstrichtupfers von dessen erstem, verkürzten in dessen zweiten, für die Probennahme geeigneten, längeren Zustand nicht erforderlich. Damit erfüllt der erfindungsgemäße Abstrichtupfer sowohl die Anforderung an einen kompakten Lagerzustand vor Gebrauch als auch einen sicheren, hygienischen und sterilen Gebrauch.

Die Erfindung ist weiterhin definiert als ein Gebinde nach Anspruch 1 aus einem Abstrichtupfer und einer Hülle für den Abstrichtupfer, wobei der Schaft in dem zweiten, für eine Probennahme geeigneten Zustand länger ist als in dem ersten Zustand, in dem der Schaft in die Hülle passt.

Vorzugsweise weist der Schaft wenigstens zwei relativ zueinander bewegliche Längsabschnitte auf, die in einerzweiten Relativposition den zweiten, für die Probennahme vorgesehenen Zustand des Schafts definieren, und die in einer ersten Relativposition den ersten, für die Unterbringung in der Hülle vorgesehenen Zustand des Schafts definieren.

Die relative Beweglichkeit der Längsabschnitte des Schafts relativ zueinander kann beispielsweise durch einen teleskopartigen Aufbau des Schafts bewirkt sein, so dass der Schaft teleskopartig ausziehbar ist.

Alternativ oder zusätzlich kann der Schaft auch bieg- oder faltbar sein.

Gemäß einer bevorzugten Ausführungsvariante des Gebindes sind Hülle und Abstrichtupfer so gestaltet, dass der Schaft selbsttätig den zweiten Zustand annimmt, wenn er aus der Hülle entnommen wird. Hierzu kann beispielsweise eine Hülle vorgesehen sein, die eine leicht klemmende Aufnahme für dasjenige Ende des Schafts aufweist, das zum Aufnehmen einer Probe ausgebildet ist. Der Schaft selbst kann hierbei teleskopartig ausgebildet sein. Die klemmende Aufnahme der Hülle ist so gestaltet, dass die Klemm- oder Haltekraft, die die klemmende Aufnahme auf das distale Ende des Schafts und/oder einen Wattebausch oder Schwamm am distalen Ende des Schafts ausübt, größer ist, als diejenige Kraft, die zum beispielsweise teleskopartigen Verlängern des Schafts erforderlich ist. Damit ist sichergestellt, dass sich beim Entnehmen des Abstrichtupfers aus der Hülle zunächst der Schaft verlängert und erst, wenn dieser maximal verlängert ist über den Schaft eine Kraft auf das distale Ende des Schafts ausgeübt werden kann, die die Klemm- oder Haltekraft übersteigt und somit eine vollständige Entnahme des Abstrichtupfers aus der Hülle bewirkt.

Die Hülle weist vorzugsweise zwei Längsenden auf, ein erstes, das die leicht klemmende Aufnahme aufweist und ein zweites Längsende, das zum Öffnen der Hülle vorgesehen ist. Wird die Hülle an diesem Ende geöffnet, kann ein Nutzer an dem Schaft ziehen, um den Abstrichtupfer aus der Hülle zu entnehmen. Dabei verlängert sich der Schaft teleskopartig und sobald er den zweiten Zustand erreicht hat, wird das zum Aufnehmen einer Probe vorgesehene Ende aus der leicht klemmenden Halterung der Hülle gezogen.

Gemäß einer ersten alternativen Variante weist der Schaft wenigstens zwei Längsabschnitte auf, die ineinandergesteckt werden können, wobei die beiden Längsabschnitte des Schafts auch im nicht ineinandergesteckten Zustand über ein Zugelement miteinander verbunden sind. Das Zugelement ist so ausgebildet und angeordnet, dass es zwischen den beiden Längsabschnitten eine Zugkraft bewirkt, die ein Ineinanderstecken der beiden Längsabschnitte des Schafts bewirkt. Beispielsweise können beide Längsabschnitte des Schafts hohl sein und in den Hohlräumen der Schaftlängsabschnitte kann ein Gummizug als Zugelement vorgesehen sein. Auch in diesem Fall kann der Abstrichtupfer beim Entnehmen aus der Hülle in einem Abschnitt des Schafts gegriffen werden, der von dem zum Aufnehmen einer Probe vorgesehenen Ende des Schafts entfernt ist. Die beiden zunächst nicht ineinandergesteckten Längsabschnitte des Schafts können durch Schwenken des einen Längsabschnitts des Schafts mithilfe des Zugelementes dazu gebracht werden, dass sich die beiden Längsabschnitte des Schafts zueinander ausrichten und selbsttätig ineinanderstecken.

Gemäß einer zweiten alternativen Variante weist der Schaft zwei Längsabschnitte auf, die über einen elastischen, vergleichsweise biegeweicheren Längsabschnitt miteinander verbunden sind, so dass der Schaft in dem elastischen Längsabschnitt leicht gebogen oder gefaltet werden kann, während die beiden an den elastischen Längsabschnitt angrenzenden Längsabschnitte ausreichend steif für die Probennahme sind. Der Schaft kann ein Lumen aufweisen, in dem ein Versteifungselement längsverschieblich angeordnet sein kann. Die Länge des Versteifungselements kann so bemessen sein, dass das Versteifungselement vom proximalen Ende des Schafts her bis in den elastischen Längsabschnitt vorgeschoben werden kann, um diesen für die Probennahme zu versteifen. Auch hierfür müssen der Schaft und das Versteifungselement nur am proximalen Ende des Abstrichtupfers gegriffen werden, so dass eine Kontamination des für Probennahme vorgesehenen Endes vermieden werden kann. Der vergleichsweise biegeweichere Längsabschnitt des Schafts ist vorzugsweise elastisch, so dass er ein selbsttätiges Strecken des Schafts bewirkt, sobald der Abstrichtupfer aus der Hülle entnommen ist.

Die Erfindung soll nun anhand von Ausführungsbeispielen mit Bezug auf die Figuren näher erläutert werden. Von den Figuren zeigt
- Figur 1:: eine erste Ausführungsform eines Abstrichtupfers gemäß der Erfindung;
- Figur 2:: den Abstrichtupfer aus Figur 1 nebst zugehöriger Hülle;
- Figur 3:: den Abstrichtupfer aus Figur 1 in einer alternativen Hülle;
- Figur 4:: eine erste alternative Variante eines Abstrichtupfers gemäß der Erfindung;
- Figur 5:: die erste alternative Variante eines Abstrichtupfers in einer Folienverpackung;
- Figur 6:: eine nicht erfindungsgemässe, dem Verständnis dienende Abwandlung der ersten alternativen Variante eines Abstrichtupfers in einer Folienverpackung
- Figur 7:: eine zweite alternative Variante eines Abstrichtupfers gemäß der Erfindung; und
- Figur 8:: die zweite alternative Variante eines Abstrichtupfers in einer Folienverpackung.

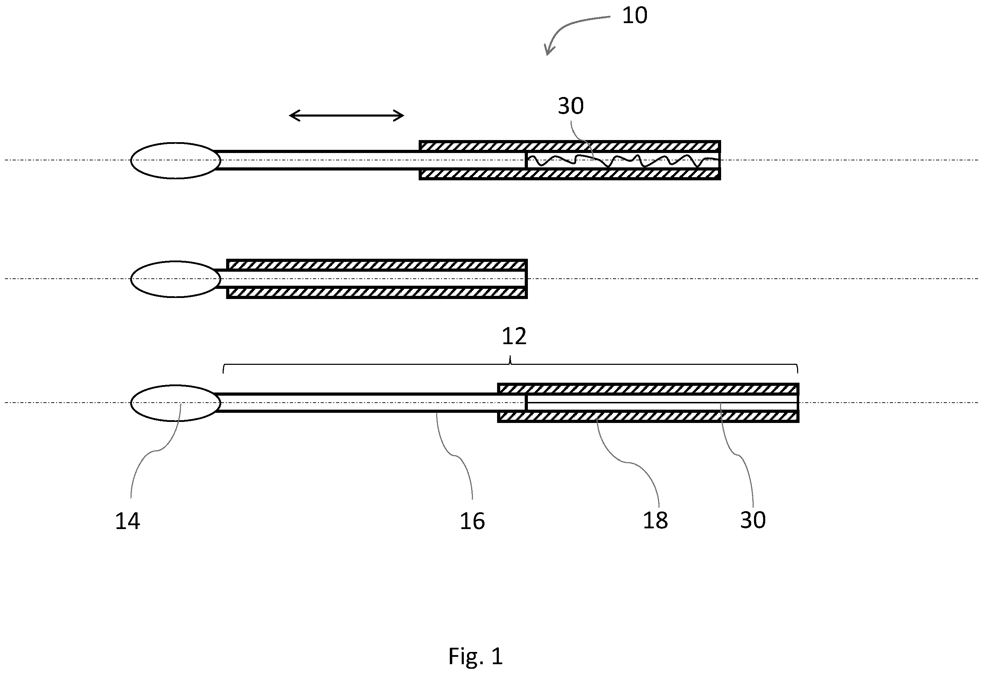

Ein Abstrichtupfer 10 weist typischerweise einen Schaft 12 auf, an dessen einem Ende beispielsweise ein Wattebausch 14 zur Probennahme angebracht ist; siehe Figur 1. Anstelle des Wattebauschs 14 kann auch ein Schwämmchen oder ein anderes, vorzugsweise weiches Probensammelelement vorgesehen sein. Wenn der Abstrichtupfer 10 zur Probennahme im Rachenraum eines Menschen gedacht ist, muss der Schaft 12 entsprechend lang sein. Dies ist für den Transport oder die Lagerung ungünstig. Entsprechend weist der Abstrichtupfer 10 einen Schaft 12 auf, der zwei Längsabschnitte 16 und 18 aufweist, die relativ zueinander beweglich sind. In dem in Figur 1 abgebildeten Ausführungsbeispiel sind die Längsabschnitte 16 und 18 des Schafts 12 relativ zueinander teleskopartig verschieblich. Dadurch ist es möglich, dass der Schaft 12 des Abstrichtupfers 10 wenigstens zwei Zustände einnehmen kann, nämlich einen ersten, ineinandergeschobenen Zustand, wie er in Figur 1b abgebildet ist, und einen zweiten, voll ausgezogenen Zustand, wie er in Figur 1c abgebildet ist. Natürlich sind auch Zwischenzustände möglich, wie beispielsweise Figur 1a zeigt. Der in Figur 1c abgebildete, voll ausgezogene, zweite Zustand des Abstrichtupfers 10 führt dazu, dass der Schaft 12 entsprechend lang ist und beispielsweise ausreicht, um eine Probe im Rachenraum eines Menschen zu nehmen.

Bei einem teleskopartig ausziehbaren Abstrichtupfer besteht grundsätzlich das Problem, dass der Abstrichtupfer zum Auseinanderziehen der beiden teleskopartig relativ zueinander verschieblichen Längsabschnitte 16 und 18 des Schafts auch im Bereich des für die Probennahme vorgesehenen Endes mit dem Wattebausch 14 angefasst werden muss. Damit könnte das für die Probennahme vorgesehene Ende des Abstrichtupfers 10 kontaminiert werden.

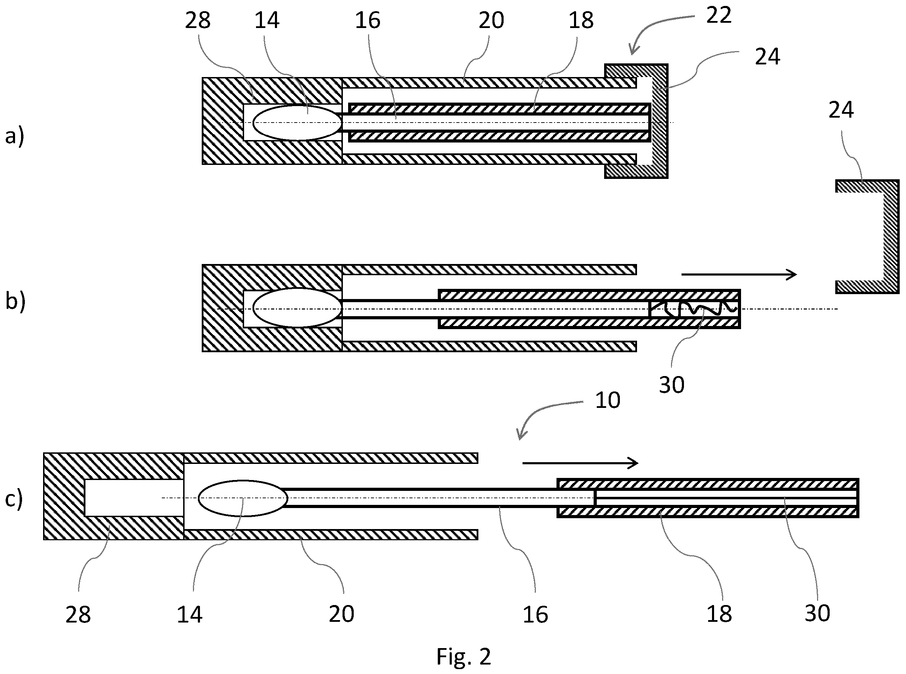

Daher ist eine Hülle 20 vorgesehen, die den Abstrichtupfer 10 aufnehmen kann; siehe Figur 2. Die Hülle 20 kann an einem Ende 22 geöffnet werden. Hierzu ist eine abnehmbare Kappe 24 vorgesehen. Am gegenüberliegenden Ende 26 der Hülle 20 ist eine klemmende Aufnahme 28 für das für die Probennahme vorgesehene Ende des Abstrichtupfers - also beispielsweise für den Wattebausch 14 - vorgesehen.

Um den Abstrichtupfer 10 zu benutzen, muss zunächst die Kappe 24 der Hülle 20 entfernt werden. Anschließend kann ein Nutzer den Längsabschnitt 18 des Schafts an dessen proximalen Ende greifen und aus der Hülle 20 ziehen. Solange der Schaft 12 noch nicht seinen vollständig ausgezogenen Zustand erreicht hat, gleitet der zweite, proximale Längsabschnitt 18 des Schafts über den ersten, distalen Längsabschnitt 16 des Schafts, wobei der Wattebausch 14 von der klemmenden Aufnahme 28 der Hülle 20 gehalten wird. Die durch den Klemmabschnitt 28 bewirkte Klemmkraft ist so eingestellt, dass sie größer ist, als die beim Verschieben der Längsabschnitte 16 und 18 wirkende Reibung. Sobald der Schaft 12 seine vollständig ausgezogene Länge erreicht hat, wird auch der distale Längsabschnitt 16 des Schafts 12 und mit ihm der Wattebausch 14 aus der klemmenden Aufnahme 28 und der Hülle 20 herausgezogen; siehe Figur 2c. Damit der zweite, proximale Längsabschnitt 18 nicht vollständig von dem ersten, distalen Längsabschnitt 16 des Schafts 12 abgezogen wird, ist beispielsweise ein Anschlag vorgesehen (nicht gezeigt) oder auch ein Faden 30, der zunächst locker ist und mit dem Auseinanderziehen der Längsabschnitte 16 und 18 gespannt wird. Der Faden 30 ist vollständig gespannt, bevor der zweite, proximale Längsabschnitt 18 von dem ersten, distalen Längsabschnitt 16 abgezogen ist.

Die klemmende Aufnahme 28 stellt somit sicher, dass ein Nutzer das für die Probennahme vorgesehene Ende des Abstrichtupfers 10 nicht zu berühren braucht, um den Abstrichtupfer 10 auf seine maximale Länge auseinanderzuziehen.

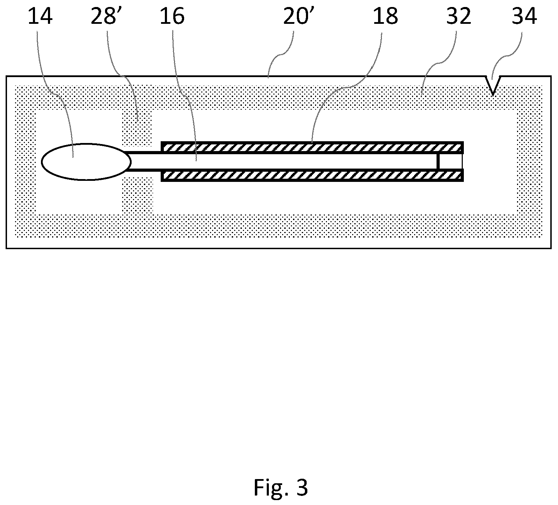

Anstelle der in Figur 2 abgebildeten Hülle kann auch eine Hülle 20' vorgesehen sein, die von zwei miteinander verschweißten oder verklebten Folien gebildet ist, wie dies in Figur 3 abgebildet ist. Die Verklebung oder Verschweißung 32 ist durch eine Musterung in Figur 3 angedeutet. Die Folienverpackung 20' kann auf der dem Wattebausch 14 abgewandten Seite aufgerissen werden; hierzu ist eine Kerbe 34 in der Hülle 20' vorgesehen. Ein Nutzer kann dann mit einer Hand das proximale Ende des zweiten, proximalen Längsabschnitts 18 des Schafts 12 greifen und mit der anderen Hand die Hülle 20' im Bereich des Wattebauschs 14 greifen, um den Wattebach 14 durch die Hülle 20' hindurch festzuhalten, um den Schaft 12 auf seine volle Länge auszuziehen. Anschließend kann der Abstrichtupfer 10 vollständig aus der Hülle 20' entnommen werden. Außerdem bildet die Verschweißung 32 eine Engstelle 28', die den Wattebausch 14 so lange zurückhält, bis der Schaft 12 vollständig ausgezogen ist.

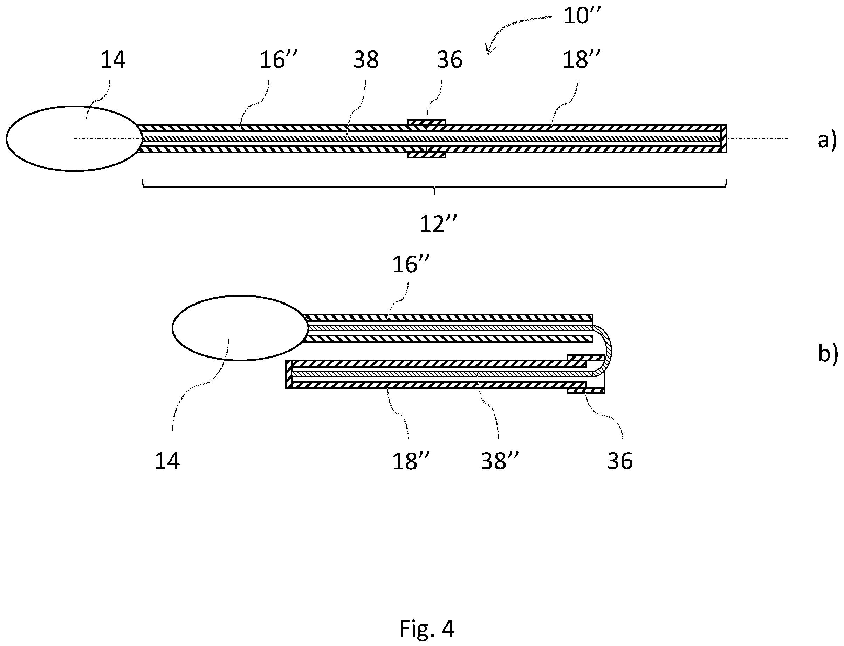

Anstelle eines teleskopartigen Schafts 12 kann auch ein Schaft 12" vorgesehen sein, der zwei ineinander steckbare Längsabschnitte 16" und 18" aufweist, wie dies in Figur 4 abgebildet ist. Die beiden Längsabschnitte 16" und 18" können durch Lösen der Steckverbindung 36 voneinander getrennt werden und dann beispielsweise so relativ zueinander positioniert werden wie dies in Figur 3b abgebildet ist.

Um den Abstrichtupfer 10" aus dem in Figur 4b abgebildeten ersten Zustand in den in Figur 3a abgebildeten zweiten Zustand zu überführen, ist ein Zugelement 38, beispielsweise ein Gummizug, vorgesehen, der sich im Inneren der beiden Längsabschnitte 16" und 18" des Schafts 12" erstreckt und mit einer gewissen Zugkraft gespannt ist. Die Zugkraft des Zugelements 38 führt dazu, dass ein Nutzer nur den proximalen Längsabschnitt 18" des Schafts 12" anzufassen braucht, um den Abstrichtupfer 10" aus dem in Figur 3b abgebildeten ersten Zustand in den in Figur 4a abgebildeten zweiten Zustand zu überführen. Infolge der Zugkraft des Zugelementes 38 reicht es nämlich, den proximalen Längsabschnitt 18" mit dessen Öffnung nach unten in Richtung der Erdanziehungskraft weisen zu lassen, sodass sich die beiden Längsabschnitte 18" und 16" infolge der Erdanziehungskraft annähernd längs zueinander ausrichten. Die Zugkraft des Zugelementes 38 führt dann dazu, dass der distale Längsabschnitt 16" des Schafts 12" in die Steckverbindung 36 hineingezogen wird, sodass die beiden Längsabschnitte 16" und 18" im Ergebnis ineinandergesteckt sind, ohne dass ein Nutzer hierzu den distalen Längsabschnitt 16" oder den Wattebausch 14 zu berühren braucht.

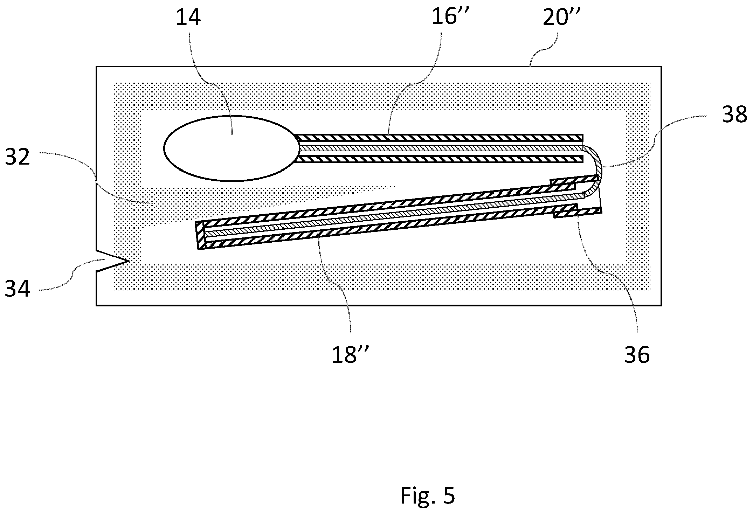

Auch der Abstrichtupfer 10" gemäß Figur 4 kann in einer Hülle 20", beispielsweise einer Folienverpackung, die von zwei miteinander verschweißten oder verklebten Folien gebildet ist, verpackt sein und ein entsprechendes Gebinde bilden; siehe Figur 5. Die Folienverpackung 20" ist so gestaltet, dass die Verschweißung 32 und die Anordnung der Kerbe 34 es erlauben, die Folienverpackung 20" so öffnen, dass zunächst nur der proximale Längsabschnitt 18" des Abstrichtupfers 10" freiliegt und gegriffen werden kann, während der distale Längsabschnitt 18" des Abstrichtupfers 10" mit dem Wattebauch 14 nach wie vor geschützt ist.

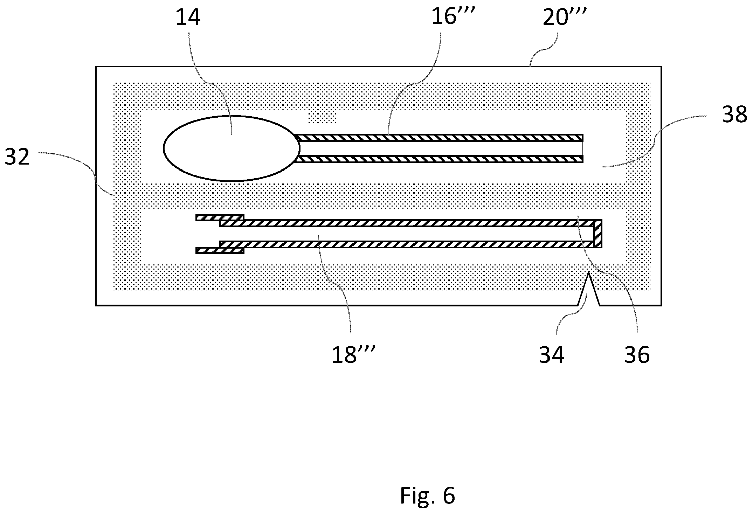

In einer vereinfachten nicht erfindungsgemäßen, dem Verständnis dienenden Abwandlung des Ausführungsbeispiels aus den Figuren 4 und 5 kann das Zugelement 38 auch weggelassen werden, so dass die Schaftteile 16"' und 18‴ zunächst nicht miteinander verbunden sind und für den Gebrauch zusammengesteckt werden können. In diesem Fall ist eine Folienverpackung als Hülle mit zwei Kammern hilfreich; siehe Figur 6.

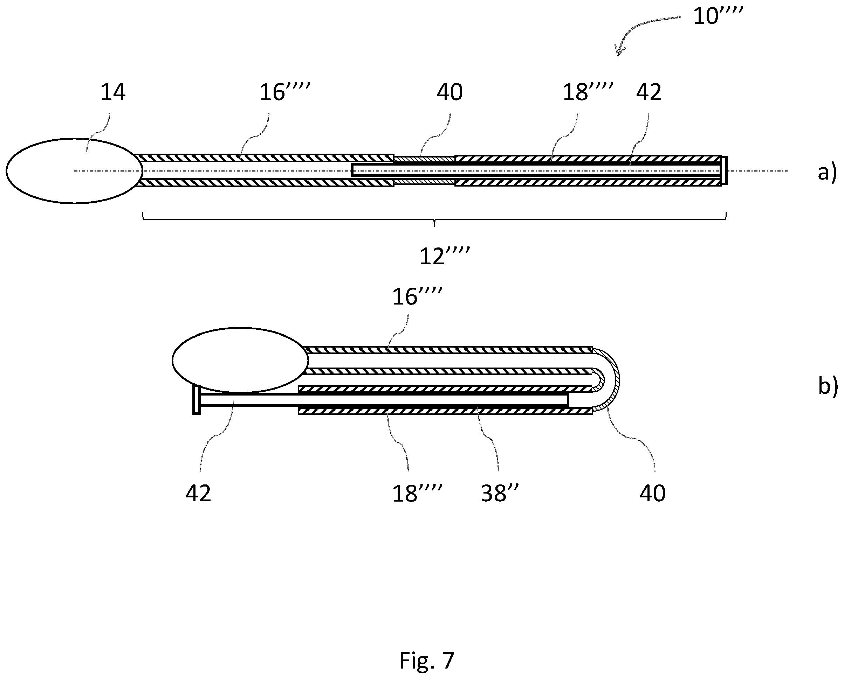

Gemäß einer zweiten alternativen Variante weist der Schaft 12ʺʺ zwei Längsabschnitte 16ʺʺ und 18ʺʺ auf, die über einen elastischen, vergleichsweise biegeweicheren Längsabschnitt 40 miteinander verbunden sind, so dass der Schaft 12ʺʺ in dem elastischen Längsabschnitt 40 leicht gebogen oder gefaltet werden kann, während die beiden an den elastischen Längsabschnitt 40 angrenzenden Längsabschnitte 16ʺʺ und 18ʺʺ ausreichend steif für die Probennahme sind; siehe Figur 7a.

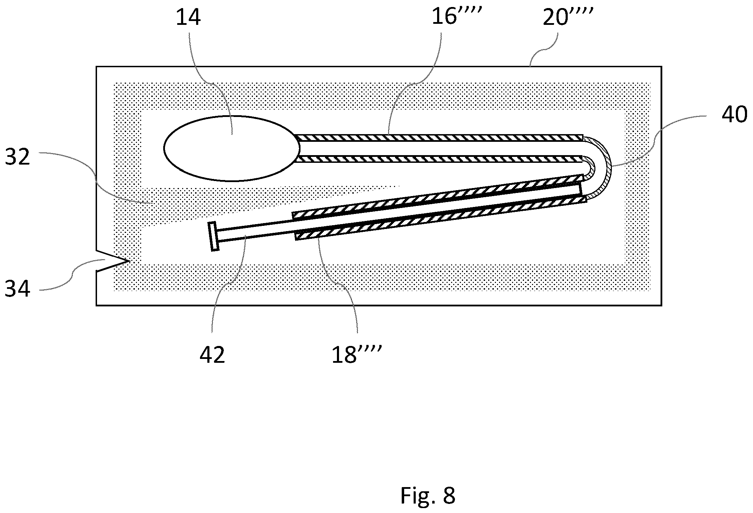

Um den Schaft für den Gebrauch zu versteifen, weist der Schaft ein Lumen auf, in dem ein Versteifungselement 42 längsverschieblich angeordnet ist. Die Länge des Versteifungselements 42 ist so bemessen, dass das Versteifungselement 42 vom proximalen Ende des Schafts 12‴ her bis in den elastischen Längsabschnitt 40 vorgeschoben werden kann, um den elastischen Längsabschnitt 40 für die Probennahme zu versteifen; siehe Figur 7a. Falls der elastische Längsabschnitt 40 so gestaltet ist und aus einem Material besteht, dass der elastische Längsabschnitt 40 zwar elastisch gebogen werden kann, wie dies in Figuren 7b und 8 abgebildet ist, aber im gestreckten, entspannten Zustand eine ausreichende Steifigkeit besitzt, die einen sachgerechten Gebrauch des Abstrichtupfers erlaubt, kann auf ein Versteifungselement 42 auch verzichtet werden. Ein entsprechendes Ausführungsbeispiel kann ähnlich aussehen, wie das in Figur 7a abgebildete Ausführungsbeispiel, nur ohne das Versteifungselement 42.

Hierbei müssen der Schaft 12ʺʺ und das Versteifungselement 42 nur am proximalen Ende des Abstrichtupfers 10ʺʺ gegriffen werden, so dass eine Kontamination des für eine Probennahme vorgesehenen Endes 14 vermieden werden kann.

Zusätzlich besteht der elastische Längsabschnitt 40 aus flexiblem Material, das Rückstellkräfte bewirkt, welche zur Folge haben, dass sich der elastische Längsabschnitt 40 bei Relaxation automatisch so ausrichtet, dass die Längsabschnitte 16ʺʺ und 18ʺʺ parallel und hintereinander ausgerichtet sind. Die Flexibilität des gesamten Aufbaus ist so gering, dass die Anwendung als Abstrichtupfer 12ʺʺ nicht beeinträchtigt ist.

Eine Folienverpackung 20ʺʺ ähnlich derjenigen, wie sie in Figur 8 abgebildet ist, erlaubt es aufgrund der Gestaltung der Verschweißung 32 und der Anordnung der Kerbe 34 wie bei dem in Figur 5 abgebildeten Ausführungsbeispiel die Folienverpackung 20ʺʺ so öffnen, dass zunächst nur der proximale Längsabschnitt 18ʺʺ des Abstrichtupfers 10ʺʺ freiliegt und gegriffen werden kann, während der distale Längsabschnitt 18ʺʺ des Abstrichtupfers 10ʺʺ mit dem Wattebauch 14 noch in der Folienverpackung 20ʺʺ bleiben kann und so geschützt ist.

Alle Ausführungsvarianten bieten einen kompakt verpackten und gleichwohl die Anforderungen an die Hygiene erfüllenden Abstrichtupfer.

### Bezugszeichenliste:

- 10: Abstrichtupfer
- 12: Schaft
- 14: Wattebausch
- 16: distaler Längsabschnitt des Schafts
- 18: proximaler Längsabschnitt des Schafts
- 20: Hülle, Folienverpackung
- 22: Ende der Hülle
- 24: Kappe der Hülle
- 26: gegenüberliegendes Ende
- 28: klemmende Aufnahme
- 30: Faden
- 32: Verklebung oder Verschweißung
- 34: Kerbe
- 36: Steckverbindung
- 38: Zugelement
- 40: elastischer Längsabschnitt des Schafts
- 42: Versteifungselement

## Patentansprüche

1. Gebinde mit einer Hülle (20; 20'; 20"; 20ʺʺ) und einem darin befindlichen Abstrichtupfer (10; 10"; 10ʺʺ) für die Probennahme, insbesondere im Rachen eines Menschen,
wobei der Abstrichtupfer (10; 10"; 10ʺʺ) einen länglichen Schaft (12; 12"; 12ʺʺ) aufweist, der zum Halten des Abstrichtupfers (10; 10"; 10ʺʺ) dient und ein Ende aufweist, das zum Aufnehmen einer Probe ausgebildet ist, wobei der Abstrichtupfer (10; 10"; 10ʺʺ) in einem ersten verkürzten Zustand seines Schafts (12; 12"; 12ʺʺ) steril von der Hülle (20; 20'; 20"; 20ʺʺ) umschlossen ist
**dadurch gekennzeichnet, dass** die Hülle (20; 20'; 20"; 20ʺʺ) und der Abstrichtupfer (10; 10"; 10ʺʺ) so aufeinander abgestimmt gestaltet sind, dass der Schaft (12; 12"; 12ʺʺ) bei Entnahme des Abstrichtupfers aus der Hülle (20; 20'; 20"; 20ʺʺ) automatisch vom verkürzten ersten Zustand des Schafts in einen zweiten, für eine Probennahme geeigneten Zustand übergeht, in dem der Schaft (12; 12"; 12ʺʺ) länger ist als in einem ersten, in die Hülle (20; 20'; 20"; 20ʺʺ) passenden Zustand.

2. Gebinde nach Anspruch 1, **dadurch gekennzeichnet, dass** der Schaft (12; 12"; 12ʺʺ) wenigstens zwei relativ zueinander bewegliche Längsabschnitte (16, 18; 16", 18"; 16ʺʺ, 18ʺʺ) aufweist, die in einer zweiten Relativposition den zweiten, für die Probennahme vorgesehenen Zustand des Schafts (12; 12"; 12ʺʺ) definieren, und die in einer ersten Relativposition den ersten, für die Unterbringung in der Hülle (20; 20'; 20"; 20ʺʺ) vorgesehenen, kürzeren Zustand des Schafts (12; 12"; 12ʺʺ) definieren.

3. Gebinde nach Anspruch 1 oder 2, **dadurch gekennzeichnet, dass** der Schaft (12) teleskopartig ausziehbar ist.

4. Gebinde nach wenigstens einem der Ansprüche 1 bis 3, **dadurch gekennzeichnet, dass** der Schaft (12"; 12ʺʺ) bieg- oder faltbar ist.

5. Gebinde nach wenigstens einem der Ansprüche 1 bis 4, **dadurch gekennzeichnet, dass** die Hülle (20) eine Haltkraft bewirkende Aufnahme (28; 28') für dasjenige Ende des Schafts aufweist, das zum Aufnehmen einer Probe ausgebildet ist.

6. Gebinde nach Anspruch 5, **dadurch gekennzeichnet, dass** die die Haltkraft bewirkende Aufnahme der Hülle ist so gestaltet, dass die Klemm- oder Haltekraft, die die Haltkraft bewirkende Aufnahme auf das distale Ende des Schafts und/oder einen Wattebausch oder Schwamm am distalen Ende des Schafts ausübt, größer ist, als diejenige Kraft, die zum Verlängern des Schafts erforderlich ist

7. Gebinde nach wenigstens einem der Ansprüche 1 bis 6, **dadurch gekennzeichnet, dass** die Hülle (20; 20') zwei Längsenden aufweist, nämlich ein erstes Längsende, das die leicht klemmende Aufnahme (28; 28') aufweist und ein zweites Längsende, das zum Öffnen der Hülle vorgesehen ist, wobei sich das zum Halten des Abstrichtupfers (10; 10"; 10ʺʺ) dienende, proximale Ende des Schafts (12; 12') nahe dem zweiten, zum Öffnen vorgesehenen Längsende der Hülle (20; 20') befindet.

8. Gebinde nach wenigstens einem der Ansprüche 1 bis 7, **dadurch gekennzeichnet, dass** der Schaft (12") wenigstens zwei Längsabschnitte (16", 18") aufweist, die ineinandergesteckt werden können, wobei die beiden Längsabschnitte (16", 18") des Schafts (12") auch im nicht ineinandergesteckten Zustand über ein Zugelement miteinander verbunden und das Zugelement ist so ausgebildet und angeordnet, dass es zwischen den beiden Längsabschnitten eine Zugkraft bewirkt, die ein Ineinander stecken der beiden Längsabschnitte des Schafts bewirkt.

9. Gebinde nach wenigstens einem der Ansprüche 1 bis 8, **dadurch gekennzeichnet, dass** der Schaft (12ʺʺ) wenigstens zwei Längsabschnitte (16"", 18"") aufweist, die über einen elastischen, vergleichsweise biegeweicheren Längsabschnitt (40) miteinander verbunden sind, so dass der Schaft (12‴) in dem elastischen Längsabschnitt (40) leicht gebogen oder gefaltet werden kann, während die beiden an den elastischen Längsabschnitt (40) angrenzenden Längsabschnitte (16ʺʺ, 18ʺʺ) ausreichend steif für die Probennahme sind.

10. Gebinde nach Anspruch 9, **dadurch gekennzeichnet, dass** der Schaft (12ʺʺ) ein Lumen aufweist, in dem ein Versteifungselement (42) derart längsverschieblich angeordnet ist, dass das Versteifungselement (42) vom proximalen Ende des Schafts (12‴) her bis in den elastischen Längsabschnitt (40) vorgeschoben werden kann, um diesen für die Probennahme zu versteifen.

11. Abstrichtupfer (10; 10"; 10ʺʺ) für die Probennahme, insbesondere im Rachen eines Menschen, wobei der Abstrichtupfer (10; 10"; 10ʺʺ) einen länglichen Schaft (12; 12"; 12ʺʺ), der zum Halten des Abstrichtupfers (10; 10"; 10ʺʺ) dient und ein Ende aufweist, das zum Aufnehmen einer Probe ausgebildet ist, wobei der Schaft (12; 12"; 12ʺʺ) einen zweiten, für eine Probennahme geeigneten Zustand annehmen kann, in dem der Schaft (12; 12"; 12ʺʺ) länger ist als in einem ersten, in eine Hülle (20; 20'; 20"; 20ʺʺ) passenden Zustand, **dadurch gekennzeichnet, dass**
der Schaft wenigstens einen elastischen Bestandteil (38; 40) aufweist, der einen selbsttätigen Übergang von dem ersten in den zweiten Zustand bewirkt.

## Claims

1. Assembly comprising a sleeve (20; 20'; 20"; 20ʺʺ) and a swab (10; 10"; 10ʺʺ) located therein, for taking samples, in particular from the human pharynx,
in which the swab (10; 10"; 10ʺʺ) has a longitudinal shaft (12; 12"; 12ʺʺ) which serves to hold the swab (10; 10"; 10ʺʺ) and has an end which is designed for taking a sample, wherein the swab (10; 10"; 10ʺʺ) is sterily enclosed by the sleeve (20; 20'; 20"; 20ʺʺ) in a first shortened state of its shaft (12; 12"; 12ʺʺ),
**characterised in that** the sleeve (20; 20'; 20"; 20ʺʺ) and the swab (10; 10"; 10ʺʺ) are shaped to fit each other, so that the shaft (12; 12"; 12ʺʺ) automatically changes from a first shortened state of the shaft to a second state suitable for sample taking when the swab is removed from the sleeve (20; 20'; 20"; 20‴ʺ), in which the shaft (12; 12"; 12ʺʺ) is longer than in a first state fitting into the sleeve (20; 20'; 20"; 20ʺʺ).

2. Assembly according to claim 1, **characterised in that** the shaft (12; 12"; 12ʺʺ) has at least two longitudinal sections (16, 18; 16", 18"; 16ʺʺ, 18ʺʺ) that are movable relative to each other, which, in a second relative position, define the second state of the shaft (12; 12"; 12ʺʺ) intended for sample taking and which, in a first relative position, define the shorter state of the shaft (12; 12"; 12ʺʺ) intended for reception in the sleeve (20; 20'; 20"; 20ʺʺ).

3. Assembly according to claim 1 or 2, **characterised in that** the shaft (12) can be extended telescopically.

4. Assembly according to at least one of claims 1 to 3, **characterised in that** the shaft (12"; 12ʺʺ) is bendable or foldable.

5. Assembly according to at least one of claims 1 to 4, **characterised in that** the sleeve (20) has a receptacle (28; 28') for the end of the shaft, which is designed for taking a sample, causing a holding force.

6. Assembly according to claim 5, **characterised in that** the receptacle of the sleeve causing the holding force is shaped such that the clamping or holding force exerted by the receptacle causing the holding force on the distal end of the shaft and/or or on a cotton wool pad or foam at the distal end of the shaft, is greater than the force required to extend the shaft.

7. Assembly according to at least one of claims 1 to 6, **characterised in that** the sleeve (20; 20') has two longitudinal ends, namely a first longitudinal end, which has the slightly clamping receptacle (28; 28'), and a second longitudinal end, which is provided for opening the sleeve, wherein the proximal end of the shaft (12; 12'), serving to hold the swab (10; 10"; 10ʺʺ), is located near the second longitudinal end of the sleeve (20; 20') provided for opening.

8. Assembly according to at least one of claims 1 to 7, **characterised in that** the shaft (12") has at least two longitudinal sections (16", 18") which can be inserted into one another, wherein the two longitudinal sections (16", 18") of the shaft (12") are also connected to each other in the non-interlocked state by a tension element, and the tension element is designed and arranged in such a way that it causes a tensile force between the two longitudinal sections, which causes the two longitudinal sections to interlock.

9. Assembly according to at least one of claims 1 to 8, **characterised in that** the shaft (12ʺʺ) has at least two longitudinal sections (16ʺʺ, 18ʺʺ) which are connected to each other by a comparatively more flexible elastic longitudinal section (40) so that the shaft (12‴) can be easily bent or folded in the elastic longitudinal section (40), while the two longitudinal sections (16ʺʺ, 18ʺʺ) adjacent to the elastic longitudinal section (40) are sufficiently rigid for sample taking.

10. Assembly according to claim 9, **characterised in that** the shaft (12ʺʺ) has a lumen in which a stiffening element (42) is mounted so as to slide longitudinally, so that the stiffening element (42) can be advanced from the proximal end of the shaft (12‴) into the elastic longitudinal section (40) to stiffen it for sampling.

11. Swab (10; 10"; 10ʺʺ) for taking samples, in particular from the human pharynx, wherein the swab (10; 10"; 10ʺʺ) has a longitudinal shaft (12; 12"; 12‴ʺ) which serves to hold the swab (10; 10"; 10ʺʺ), and an end which is designed for taking a sample, wherein the shaft (12; 12"; 12‴ʺ) can assume a second state suitable for taking a sample, in which the shaft (12; 12"; 12ʺʺ) is longer than in a first state, fitting into a sleeve (20; 20'; 20"; 20ʺʺ), **characterised in that**
the shaft has at least one elastic component (38; 40) which causes an automatic transition from the first to the second state.

## Revendications

1. Assemblage comprenant une enveloppe (20 ; 20' ; 20" ; 20"") et un écouvillon (10 ; 10" ; 10""), qui s'y trouve, pour la prise d'échantillons, en particulier dans le pharynx de l'homme,
dans lequel l'écouvillon (10 ; 10" ; 10"") a un fût (12 ; 12" ; 12"") longitudinal, qui sert au maintien de l'écouvillon (10 ; 10" ; 10""), et a une extrémité, qui est constituée pour la prise d'un échantillon, dans lequel l'écouvillon (10 ; 10" ; 10"") est entouré de manière stérile de l'enveloppe (20 ; 20' ; 20" ; 20"") dans un premier état raccourci de son fût (12 ; 12" ; 12""),
**caractérisé en ce que** l'enveloppe (20 ; 20' ; 20" ; 20"") et l'écouvillon (10 ; 10" ; 10"") sont conformés en étant adaptés l'un à l'autre, de manière à ce que le fût (12 ; 12" ; 12"") passe, lors du retrait de l'écouvillon de l'enveloppe (20 ; 20' ; 20" ; 20""), automatiquement d'un premier état raccourci du fût à un deuxième état propre à une prise d'échantillon, dans lequel le fût (12 ; 12" ; 12"") est plus long que dans un premier état s'adaptant dans l'enveloppe (20 ; 20' ; 20" ; 20"").

2. Assemblage suivant la revendication 1, **caractérisé en ce que** le fût (12 ; 12" ; 12"") a au moins deux tronçons (16, 18 ; 16", 18" ; 16"", 18"") longitudinaux mobiles les uns par rapport aux autres, qui, dans une deuxième position relative, définissent le deuxième état du fût (12 ; 12" ; 12"") prévu pour la prise d'échantillon et qui, dans une première position relative, définissent l'état plus court du fût (12 ; 12" ; 12"") prévu pour la réception dans l'enveloppe (20 ; 20' ; 20" ; 20"").

3. Assemblage suivant la revendication 1 ou 2, **caractérisé en ce que** le fût (12) peut être retiré téléscopiquement.

4. Assemblage suivant au moins l'une des revendications 1 à 3, **caractérisé en ce que** le fût (12' ; 12"") est cintrable ou pliable.

5. Assemblage suivant au moins l'une des revendications 1 à 4, **caractérisé en ce que** l'enveloppe (20) a un logement (28; 28'), provoquant une force de maintien, de l'extrémité du fût, qui est constitué pour la prise d'un échantillon.

6. Assemblage suivant la revendication 5, **caractérisé en ce que** le logement de l'enveloppe provoquant la force de maintien est conformé de manière à ce que la force de serrage ou de maintien, qui exerce la réception provoquant la force de maintien sur l'extrémité distale du fût et/ou sur un tampon d'ouate ou une mousse à l'extrémité distale du fût, est plus grande que la force, qui est nécessaire pour allonger le fût.

7. Assemblage suivant au moins l'une des revendications 1 à 6, **caractérisé en ce que** l'enveloppe (20 ; 20') a deux extrémités longitudinales, à savoir une première extrémité longitudinale, qui a le logement (28 ; 28') collant facilement et une deuxième extrémité longitudinale, qui est prévue pour l'ouverture de l'enveloppe, dans lequel l'extrémité proximale du fût (12 ; 12'), servant au maintien de l'écouvillon (10 ; 10" ; 10""), se trouve près de la deuxième extrémité longitudinale, prévue pour l'ouverture, de l'enveloppe (20 ; 20').

8. Assemblage suivant au moins l'une des revendications 1 à 7, **caractérisé en ce que** le fût (12") a au moins deux tronçons (16", 18") longitudinaux, qui peuvent s'emmancher l'un dans l'autre, dans lequel les deux tronçons (16", 18") longitudinaux du fût (12") sont reliés entre eux également dans l'état non emmanché l'un dans l'autre par un élément de traction et l'élément de traction est constitué et disposé de manière à provoquer entre les deux tronçons longitudinaux une force de traction, qui provoque un emmanchement l'un dans l'autre des deux tronçons longitudinaux.

9. Assemblage suivant au moins l'une des revendications 1 à 8, **caractérisé en ce que** le fût (12"") a au moins deux tronçons (16"", 18"") longitudinaux, qui sont reliés entre eux par un tronçon (40) longitudinal élastique, comparativement de cintrage plus doux, de manière à ce que le fût (12"') puisse, dans le tronçon (40) longitudinal élastique, être courbé ou plié facilement, tandis que les deux tronçons (16"", 18"") longitudinaux voisins du tronçon (40) longitudinal élastique sont suffisamment rigides pour la prise d'échantillon.

10. Assemblage suivant la revendication 9, **caractérisé en ce que** le fût (12"") a une lumière, dans lequel est monté coulissant longitudinalement un élément (42) de raidissement, de manière à ce que l'élément (42) de raidissement puisse être avancé de l'extrémité proximale du fût (12‴) jusque dans le tronçon (40) longitudinal élastique pour rigidifier celuici pour la prise d'échantillon.

11. Ecouvillon (10; 10"; 10"") pour la prise d'échantillon, en particulier dans le pharynx de l'homme, dans lequel l'écouvillon (10; 10"; 10"") a un fût (12 ; 12" ; 12"") longitudinal, qui sert au maintien de l'écouvillon (10; 10"; 10""), et une extrémité, qui est constituée pour la prise d'un échantillon, dans lequel le fût (12 ; 12" ; 12"") de prendre un deuxième état propre à une prise d'échantillon, dans lequel le fût (12; 12"; 12"") est plus long que dans un premier état s'adaptant dans une enveloppe (20; 20'; 20"; 20""), **caractérisé en ce que**
le fût a au moins une partie (38; 40) constitutive élastique, qui provoque un passage automatique du premier au deuxième état.
